# EUROPEAN PATENT APPLICATION

(11) **EP 0 999 194 A2**
(43) Date of publication of application: **10.05.2000**
(21) Application number: 99650101.1
(22) Date of filing: 02.11.1999
(51) Int. Cl.: C05F 9/04, C05F 17/02, C05F 7/00, C05F 9/00

(54) **Waste treatment system**

(30) Priority: 02.11.1998 IE 980905
(71) Applicant: Glas Anois Reo, Cork (IE)
(72) Inventor: O'Callaghan, Michael, Macroom, Co Cork (IE); Walsh, Andrew Richard, Cobh, Co Cork (IE); Browne, Henry, Macroom, Co Cork (IE)
(74) Representative: Gates, Marie Christina Esther

(57) **Abstract**

A method and apparatus for the treatment of waste organic material is described. The apparatus comprises a housing having bedding material adapted to sustain a population of worms. Organic material is applied (61) from a sludge settlement tank (200) to the bedding material. The sludge material is applied continually, and the bedding material and resultant worm castings are harvested at most once every 6 months. In preferred embodiments the bedding material is enclosed within a bedding enclosure (108), and the bedding enclosures (108) are removeable to effect harvesting. The environmental parameters of the housing are preferably controlled, and control means (106) may be incorporated to automate this procedure.

## Description

### Field of the Invention

The present invention relates to the treatment of waste organic material, examples of which may include industrial or household organic waste and sewage sludge. The invention is especially directed to the treatment of waste materials using biological means and in particular, to the use of a controlled environment and vermicomposting for the treatment of waste materials.

### Description of the Prior Art

In the treatment of waste organic material, various processes take place. These may involve physical, chemical or biological means, applied either singularly or in combination. For example, in the treatment of sewage sludge, there is often an initial physical separation of liquid from the sludge followed by microbial digestion of the organic fraction and a final pasteurisation or chemical dosing step to eliminate disease forming organisms. In recent years, it has been suggested that the natural ability of earthworms to breakdown waste organic material could be harnessed to achieve this treatment cycle in just one step.

The process of treating organic waste using earthworms is known as vermicomposting. The process involves the mixing and consumption of the organic material by the earthworms, followed by the subsequent composting of the residual material (castings/vermicompost/biohumus) by aerobic bacteria and fungi. This biological process is in widespread use world-wide for the treatment of organic waste. There have been a number of attempts to harness this process as a waste treatment system. For example, US Patent 4,347,632 refers to a biological toilet and organic material recycling method in which human liquid and solid waste and optionally kitchen waste may be processed using a composting container in association with the application of environmental factors which may include moisture, air, warmth, bacteria and worms. The waste material in such a system is converted into a pleasant smelling, substantially neutral, compost like plant nutritional material.

Another example is described in US Patent No 4,108,625 and relates to a system for producing fertiliser from vegetable matter using earth worms. The invention is for producing fertiliser rather than dealing with waste material specifically. It is not a continuous process in that material is not continually added. Rather, waste is sited initially and left until the worms have suitably decomposed the waste material. It also utilises a substantially uncontrolled environment except for a requirement for manual intervention to add water "every few days as required".

Other known existing technologies are generally poorly controlled, labour intensive and the input feed material often requires significant pre-treatment prior to the application of the waste to the worm beds. The most common technology employed is the simple wind-row system, where worms are maintained in long rows, drills or ricks composed of a bedding material such as wood chips, manure, green waste etc. These windrows are fed manually or by vehicles. Typically, waste organic material such as animal manure or green waste is applied to the surface of the beds at intervals and the finished vermicompost is harvested by mechanically separating the worms using sieves. These windrows are only suitable for relatively dry input feed materials as very wet slurries are not easily contained and may cause leachate run-off problems. Moreover, this system is labour intensive and open -air systems in particular will suffer from seasonal fluctuations in the vermicomposting rate.

In response to this, a number of in-vessel systems have been developed to improve performance. Domestic worm bins are among the simplest. These are often just ordinary boxes containing worms and worm bedding to which food scraps and organic yard debris are added. More sophisticated designs incorporate heating elements, insulation and leachate drains to improve performance. Nevertheless, they still have to be fed manually with pre-shredded food and are mostly designed to cater for small-scale applications, e.g. single households.

At larger scales, the "continuous flow worm reactor" is the most common engineering design used for the treatment of organic waste using earthworms. This system typically consists of worm bedding that sits on a raised mesh floor within a long trough, e.g. the raised cage reactor as produced by the Vermitech Pty, or within a self contained unit, e.g. as described in EP-A-887 328, or the composter as manufactured by Vermitech Canada and described in CA-2170924. Food, in the form of waste material, is applied to the surface of the bedding continuously and the worms migrate upwards to feed on it. The lower layers in contact with the mesh floor therefore become free of worms and this zone can be harvested. Harvesting is achieved by removal of the lower layers from the container. This is achieved by using breaker bars that disturb the lower layers, thus allowing it to fall through the mesh and into the base of the trough. Feeding is often achieved using a hopper that runs on rails along the length of the trough, thus allowing the regular application of food to the surface of the bed. A problem with such apparatus is that wet feed materials such as slurries cause problems as significant through-flow of leachate may flood the harvesting zone and collection tray. The breaker bars also become ineffectual under wet conditions. Consequently, wet feed materials such as raw sewage sludge and animal slurries must be thickened prior to application. Moreover, the regular filling of the hopper and harvesting precludes automation.

### Object of the Invention

It is an object of the present invention to provide a method and apparatus for treatment of wet sludge using natural biological processes. It is a further object of the present invention to provide an apparatus that facilitates the direct application of wet organic sludge and slurry without pre-treatment for thickening the sludge, with the harvesting of a fully stabilised finished product. It is a further object of the invention to provide such a method and apparatus that can be easily automated, obviating the need for regular manual intervention. It is a further object of the invention to provide a modular organic sludge treatment system that will achieve all three stages of sludge treatment (thickening, digestion and stabilisation) in one step. It is yet a further object to provide a system that allows for sewage sludge to be wholly treated on-site automatically.

### Brief Summary of the Invention

Accordingly the invention provides a waste treatment system for the conversion of organic sludges into worm castings comprising:
i) a housing having a support floor, walls and a roof, the housing adapted to contain a fibrous worm bedding material adapted to sustain worms,
ii) applicator means in fluid communication with a sludge settlement tank for applying sludge from the settlement tank directly to the worm bedding material wherein the applicator means is adapted to apply the sludge continually to the bedding and the bedding material being removable to effect harvesting of the castings.

The bedding material is preferably containable in a plurality of removeable bedding enclosures, the bedding enclosures being removeable to effect harvesting of the castings.

The applicator means preferably includes a pump for pumping the sludge from the settlement tank to the bedding enclosure, and spray means for spraying the sludge onto the worm bedding material, such that the sludge being sprayed is in a liquid form with less than about 5% solid material per volume.

The bedding enclosures may be adapted to be suspended within the housing, or the bedding enclosures may be mesh trays, each tray being situatable on a support floor within the housing.

The housing may comprise a plurality of floors, each floor adapted to support a plurality of bedding enclosures, and the housing may further comprise a plurality of means for spraying the sludge material onto the bedding enclosure.

The worm bedding material is adapted to provided a home for a worms, preferably red worms, and more preferably worms of the species *Eisenia fetida.*

The bedding enclosures may be provided with mounting means, suitable for mounting an additional bedding enclosure containing worm bedding material on top of an existing bedding enclosure containing worms, such that the mounting of a bedding enclosure on top of the worm containing bedding enclosure interrupts the application of sludge from the applicator means onto the worm containing bedding enclosure, and stimulates the movement of worms from the lowermost bedding enclosure to the uppermost bedding enclosure.

The bedding enclosures are preferably provided with transport means, for allowing movement of worms from a first bedding enclosure to a second bedding enclosure located above the first bedding enclosure, the transport means being located in the base of the bedding enclosures. The transport means may be apertures. Such apertures may also allow for the free passage of leachate through the fibrous bedding material, thus avoiding persistent water-logging of the worm feeding zone.

Each support floor is provided with a draining means for draining excess fluid (leachate) that filters through the bedding material, the draining means in fluid communication with a waste water treatment plant, such that excess fluid is passed from the waste treatment system to the water treatment plant.

The housing is preferably insulated.

The system further comprising means for controlling at least two environmental parameter within the enclosure, the means being responsive to at least one of moisture level and temperature. Additional environmental parameters include pH level and light level.

The invention also provides a method of converting organic sludge material to worm castings comprising the steps of:
a) providing a worm bedding material within a housing,
b) housing worms within said worm bedding material, and
c) applying organic sludge material directly from a sludge settlement tank to the bedding enclosure.

The method preferably comprises the additional step of placing the bedding material into a plurality of bedding enclosures prior to the housing of the worms in the bedding material.

Preferably sludge is applied to the surface of the bedding material in a liquid form with less than 5% solids per volume.

The method optionally comprises the additional step of mounting a second bedding enclosure on top of the first bedding enclosure, the mounting of the second bedding enclosure stimulating the movement of worms from the first bedding enclosure to the second bedding enclosure.

The method preferably comprises the additional step of removing the bedding enclosures comprising bedding material and worm castings from the housing at most once every 6 months.

According to a further aspect of the invention, there is provided a treatment system for waste material comprising:
(a) an housing containing a bed to which waste material to be treated is applied, the bed providing a habitat for worms,
(b) means for enabling passage of liquid from said bed to a soakaway region while preventing passage of solid matter from said bed to said soakaway region, and
(c) means for controlling at least two environmental parameter of the treatment system within said housing.

The bedding may additionally be contained within a suitable enclosure, the enclosure being situatable in the housing. In the waste material treatment system according to the invention, said bed may comprise at least one substantially porous and aeratable layer, said at least one layer optionally comprising substantially degradable material, e.g. wood chip, compost etc..

In the treatment system of the invention, means may be provided for reducing bulk waste material to a fine granular form in liquid suspension, before applying to the bed, said reducing means defining said means for converting waste material to a granular form.

In the preferred embodiment, the reducing means may be a shredder pump. The shredder pump may also serve as the means to move granular material in a liquid suspension to the treatment bed by pumping the granular material through a series of pipes which terminate above the bed. The pipes may terminate in a sprinkler head.

In an alternative embodiment, this means for reducing bulk waste material to granular form consists of a drum within which an arrangement of blades rotate to cut large portions of bulk material into smaller granular form.

It is further an optional feature of the invention that means may be provided to move this newly converted granular form material to the bed of the treatment system.

In one embodiment of the present invention, the means for transferring the material uses forced air to drive the granular material along a conduit to the treatment bed through an opening in the roof of the enclosure, for discharge onto the bed at selectively variable locations.

In a further variant, means are provided for directing air through the bed according to the invention for aeration of the bed. The temperature of the bed may also be controlled by forcing heated or cooled air through the bed of the system.

The invention also extends to a method of treating waste material comprising the steps of:
(a) preparing a system according to any of the herein before described aspects of the invention,
(b) loading a quantity of red worms onto the surface of the bed,
(c) allowing the worms to establish a suitable habitat in the bed ,
(d) subsequently applying waste material to the surface of the bed and,
(e) controlling at least one environmental parameter of the system.

In a method of treating waste material according to the invention, the waste material is preferably applied to the upper surface of the bed.

In a favoured embodiment of the invention, the waste material applied to the treatment system is substantially or partly comprised of sewage or other organic sludge or slurry.

### Brief Description of the Drawings

The invention will now be described in more detail having regard to the accompanying drawings, in which:
Figure 1 is a cross section through a bed prepared for application of the method according to a first embodiment of the present invention,
Figure 2 is a sectionalised pictorial view of the bed of Figure 1, when waste material is applied to it,
Figure 3 is a pictorial view of an housing and environmental control system according to a second embodiment of the present invention,
Figure 4 is a plan view of the second embodiment of the invention showing a series of bulk containers within the housing,
Figure 5 is a first side elevation of the embodiment of Figure 4,
Figure 6 is a front elevation of the embodiment of Figure 4.
Figure 7 is an elevation of the other side of the embodiment of Figure 4,
Figure 8 is a block diagram showing the incorporation of the system of the present invention into a conventional waste water treatment facility
Figure 9 is a plan view showing an apparatus for the treatment of sludge according to a third embodiment of the present invention,
Figure 10 is a side elevation of the embodiment of Figure 9 with trays mounted therein,
Figure 11 is a front elevation of the embodiment of Figure 9.

### Detailed Description of the Drawings

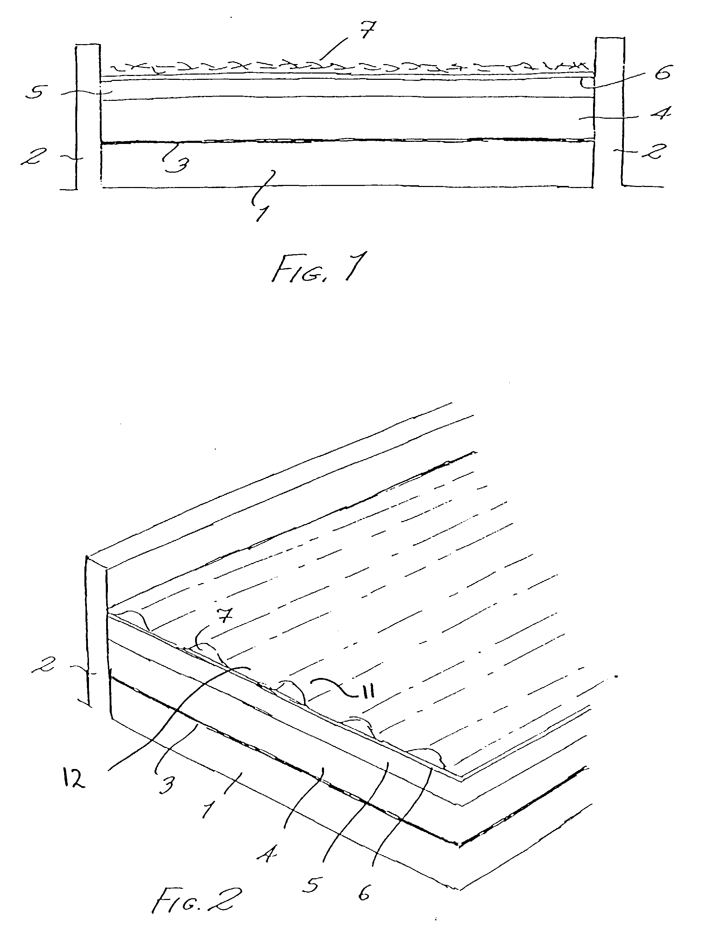

As shown in Figure 1, a bed for use in a system according to the invention is built up in a number of stages or steps. First of all, a gravel base 1 is laid in the area where the treatment is to take place for the purpose of allowing liquid to drain away through this layer of the bed. The bed is suitably contained within an enclosed generally rectangular area surrounded by a peripheral wall 2. Preferably this wall is insulated to reduce the energy required to maintain a constant temperature. The enclosure of the bed within a periphery wall restricts the run off of applied sludge material.

A layer 3 of woven porous material is then placed over the bed of gravel.

A suitable material is on the market under the name of Promisol™. This woven fabric material provides a porous structure of fine mesh, so that solids are retained above the woven layer, but water may pass through into the soakaway region defined by the gravel bed 1.

A further layer 4 of again generally porous material approximately 12 inches deep is then laid over the sheet of woven material. This layer may be substantially organic and is suitably made up of a biological material such as wood chips, but it may also comprise inorganic materials such as gravel or sand, or a mixture of various organic and inorganic substances. The purpose of this layer is to provide for aeration and soakage above the woven material so as to establish a habitat or environment for the worms which operate in the system of the invention to effect the required biological action in breaking down the waste material.

A further layer 5 of for the most part degradable material is then placed over the 12 inch bed, this further layer being approximately 4 inches in depth and consisting of compost or sawdust material, wood chips, straw, mushroom compost or chopped paper. A partial mixture of gravel and/or sand may also be included.

An optional top layer 6 may be provided by a quantity of lime or sand which is distributed lightly over the surface of the composting material 5 to provide a thin granular coating.

The worms 7 effective in the system of the invention are then spread over this formed bed and gradually move down into the bed to take up residence within it. Worms generally move away from light, so they may be encouraged to burrow into the material of the bed by being exposed to light. They burrow down into the aerated material layers 4 and 5 and form an appropriate habitat.

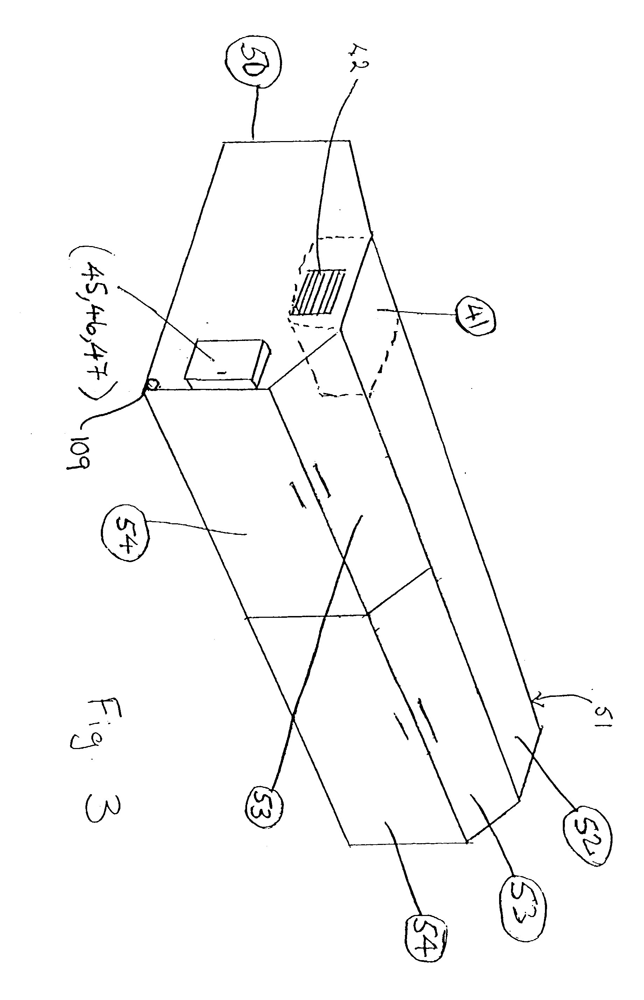

Once the worms have passed into the biological bed, a thin layer of waste material is applied over the top of the bed. The waste is preferably laid out in strips 11, in the form of agricultural drills or ridges, as shown in Figure 2, so that upstanding elongate strips 11 of waste are separated from one another by valleys 12.

The waste material may be applied directly by hand or by mechanical means.

Other materials such as peat or lime may also be deposited on the bed using the mixer feeder either separately or concurrently with the application of waste material. The addition of such material may be used to alter the pH level of the system.

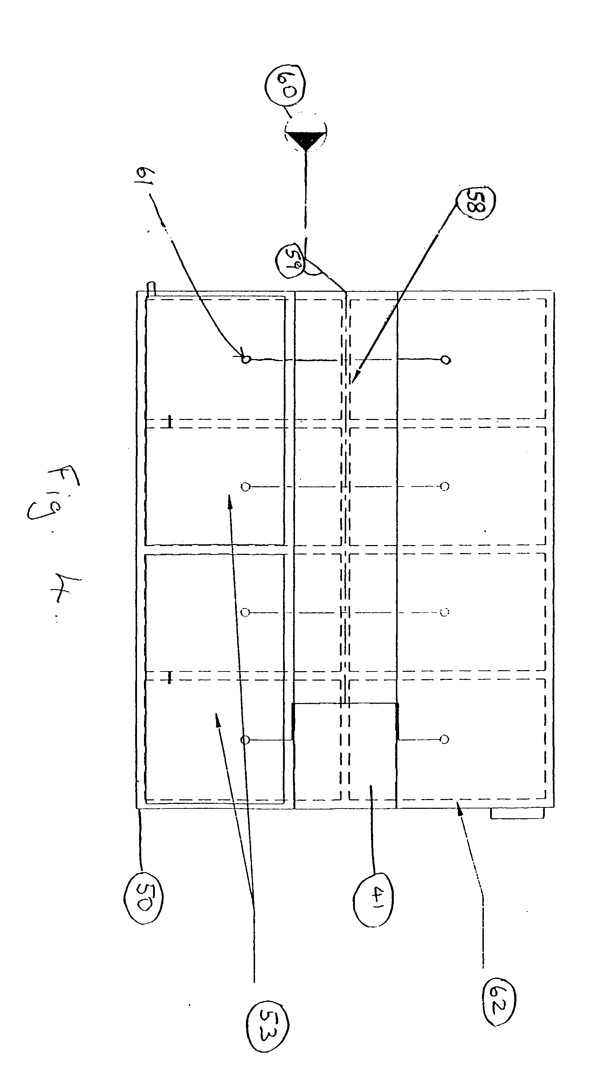

An environmental control system, which as depicted in diagramatically pictorial view in Figure 3 in use with a bed as shown in Figures 1 and 2, ensures that optimum conditions are maintained by controlling one or more environmental factors or parameters in the system. The bed or enclosure is sited within a suitable enclosure or housing 50. The housing is defined by four walls, enclosing a floor region and covered by a pitched roof. To minimise energy requirements, the housing is insulated to reduce heat transfer to or from the surrounding environment. The roof 51 of the housing 50 comprises sloping panels 53 hinged at the apex 52 of the roof so that at least one panel 53 is raisable so as to create an opening in the roof. A second set of panels 54 facilitates the opening of the housing at the side. The opening facilitates the addition of or removal from, material to and from the housing 50 from the top. This addition, or removal may be by manual or suitable mechanical means. In one embodiment, material is deposited in an automated or semi automated fashion by a conventional shredder mixer unit. This is typically a conventional shredding device of the sort used in agriculture for shredding farm produce. In addition, the housing 50 is designed so that when the panel is lowered, the housing 50 is substantially sealed so that ingress and or egress to or from the enclosure of liquids and gases is minimised. The housing 50 is adapted to include an air conditioner 41, so as to maintain the correct temperature for the worms, and also includes a leachate drain 109 to provide a fluid exit port for draining excess leachate from the housing 50.

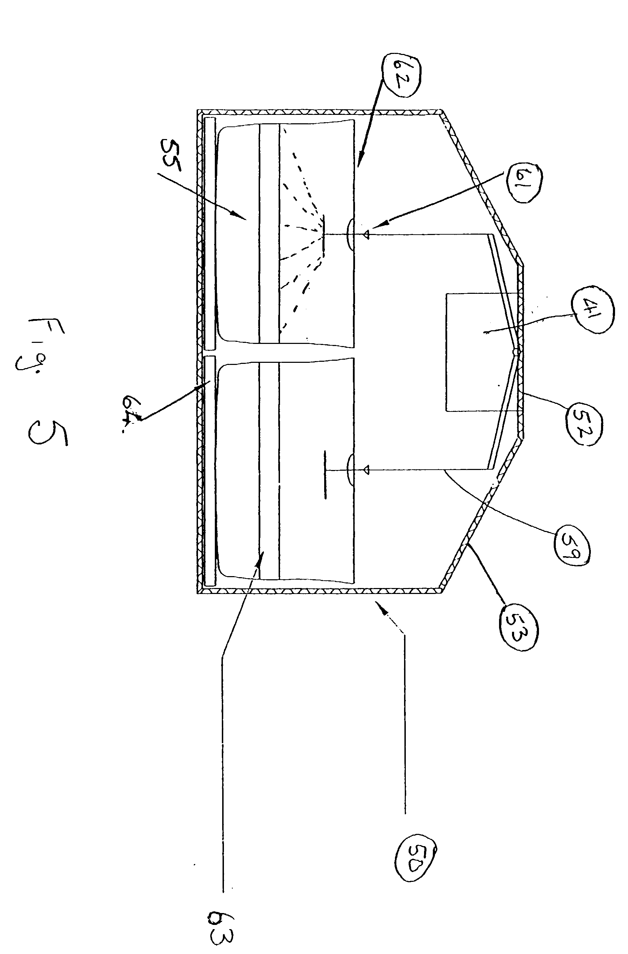

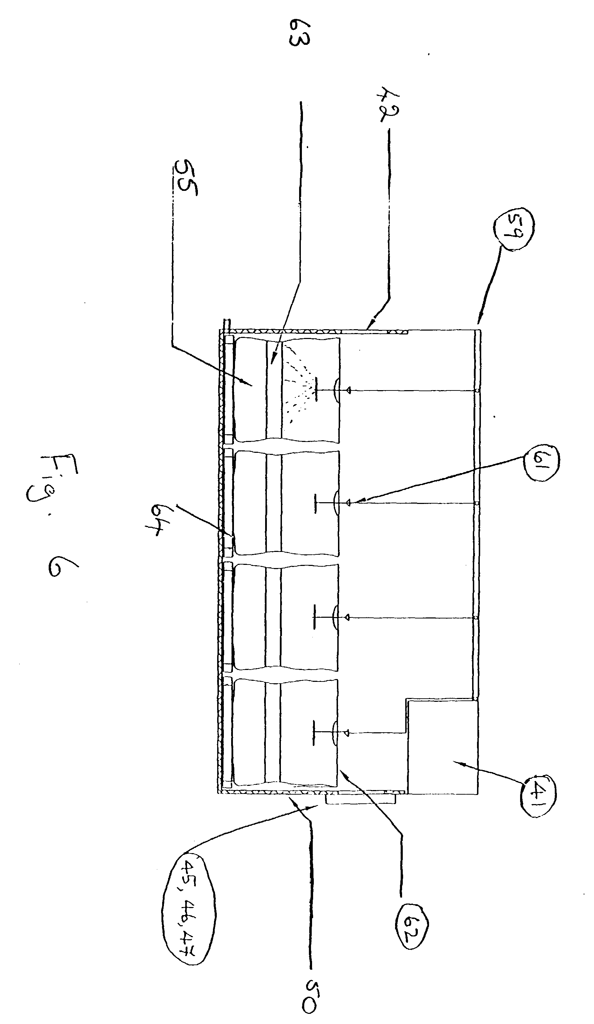

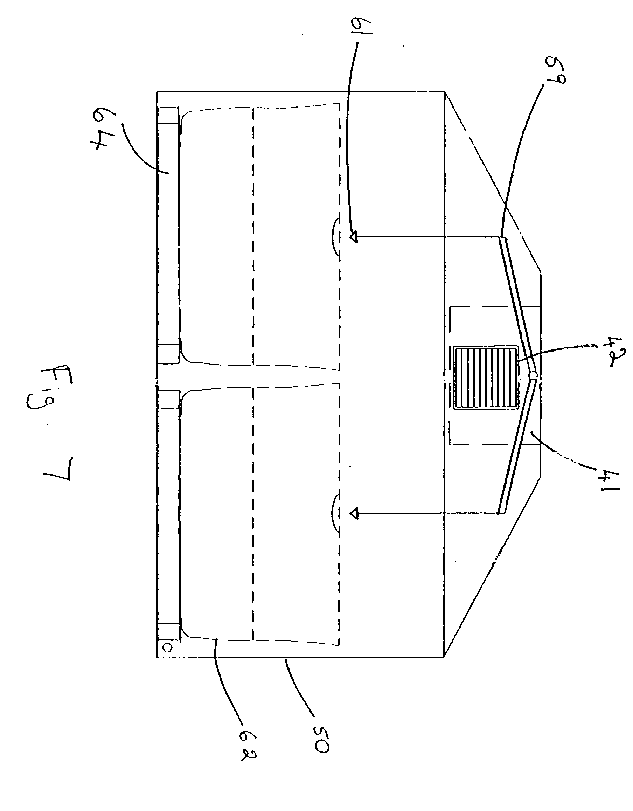

In the second embodiment of the invention, shown in Figures 4 to 7 bedding 55 is formed in a intermediate bulk container (IBC), suitably a bag 62. The bag 62 is composed of a semi-porous material, such as Promisol, which minimises the passage of solid materials. The bag 62 can either be positioned within a frame so that it is self-supporting, and/ or it can be supported, on pallets 64 or the like, within a box-like structure or the housing 50. It is possible to have any number of bags 62 within a housing as has been described above.

Worms function better if the soil or material in which they are located is aerated. Therefore, in the second embodiment the IBCs hangs above the floor of the housing 50, and air can pass between IBCs located in series within the housing 5(). The sludge material forms a layer 63 on the top of the bedding material in the IBC 62.

The air conditioner unit 41 may be used to further improve aeration in the beds. An air pump within the air conditioner takes air and blows it to the space underlying the bed or surrounding the IBC. The system is suitably partially sealed at the base so that this air is forced through the various layers of the bed to the surface and then back into the air conditioner in a continuous cycle. The conditioner may be provided with a louvered vent 42 to the atmosphere. The air conditioner may be operated on a continuous or non continuous basis depending on a number of factors, for example the amount and type of waste material, and the size of the air conditioner. The air conditioning unit may be replaced by an alternative arrangement comprising a fan or blower unit in combination with heating and cooling means.

A number of sensing means 45, 46, 47 are provided to measure environmental factors. In particular, a temperature sensor 45 is provided to measure the temperature of the bed and/or air temperature, a moisture sensor 46 is used to determine the level of humidity in the bed of the invention and pH sensors 47 are used to determine the level of alkalinity or acidity in the bed. Suitable control means compare the measured temperature with a pre set optimum level. If the measured temperature exceeds the set point, the cooling unit in the air conditioner is switched and cool air is pumped through the beds so as to effect a decrease in bed temperature until the bed temperature equals the optimum level. If the measured temperature falls below the set point then the control means switches on the heating unit in the air conditioner to effect an increase in bed temperature until the bed temperature equals the set point. The optimum temperature will depend on a variety of factors including the types of worm used, but will typically be within the range 15 to 22°.

Suitable control means also compare the pH level of the bed as measured by sensor 47 and also determine whether the pH is above or below the optimum level. A suitable acidic material, e.g. peat may be added to the bed surface either by direct application or by suitable automated means, as described herein before, if the pH is above the ideal level. Suitable alkaline material, e.g. lime may be added if the measured pH is below the ideal level, by either manual or mechanical means.

The optimum pH level will vary on the materials, methods and vermicomposting used, but will typically be pH 7, and more generally will lie within the range pH 6 to pH 8.

The moisture detector 46 with suitable control means operates a suitable water distributor e.g. a spray system, to apply water to the bed of the invention if the moisture level in the bed falls below a pre determined level.

In the embodiment shown in Figures 4 to 7, the waste material is applied directly from a sludge settlement tank, to the bed using a sprinkler system 58. Waste material, stored in the sludge settlement tank is fed through a series of pipes 59 using a shredder pump 60, which is typically of the variety known as a pyranha pump. Similarly to the shredder mixer, referenced in the first embodiment, the pyranha pump 60 is capable of shredding the waste material but also can pump the material through the pipes 59. The pipes ends in a sprinkler head 61 which is positioned above the bed to ensure an even distribution of waste material over the bed within the IBC.

An advantage of this embodiment utilising an IBC 62 over the first embodiment is that when the IBC 62 is full and must be replaced i.e. the castings have to be removed, the IBCs 62 can be removed from the frame or box 63 and replaced with a fresh set of IBCs. The IBCs 62 may be of a variety of sizes, but one tonne IBCs are preferable. The arrangement makes the removal of castings much less labour intensive, and there is no interruption of the feed take by the worms, there is a quicker turn around time in removing the castings and the removal system is not messy.

The worms digest the deposited waste material and transform it into a useful final product. A bed according to the invention, once established, will be effective for some 6 to 18 months. The digestion process effected by the worms on the waste material reduces the original material to about 25% of its original weight and volume. The final resulting material in the bed, when it comes to the end of its useful life, consists of worm casts, of significantly reduced volume and weight as compared with the quantity and weight of the material which has been applied to the system during its period of use. This final material from the bed is immediately usable as a compost and/or soil conditioner. In any event, the system of the invention enables expensive technology to be dispensed with and also eliminates any necessity to use potentially hazardous chemicals.

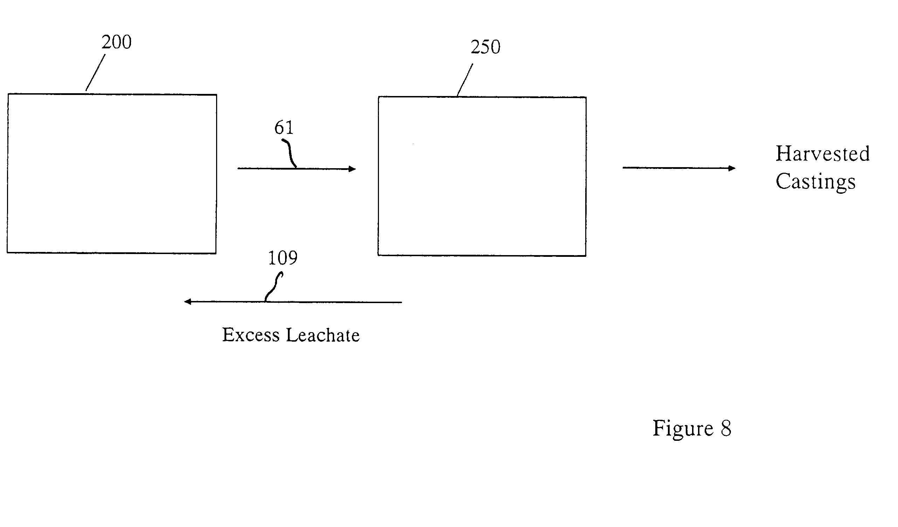

Figures 8 to 11 show a third and preferred embodiment of the present invention. The same reference numerals are used for similar components. Similarly to that described with reference to the previous embodiments, this third embodiment incorporates the use of an external housing 50.

Figure 8 shows the incorporation of the system of the present invention into a conventional waste water treatment plant 200. Sludge is taken from the sludge settlement tanks of the waste water treatment facility, passing through piping 61 directly to the system 250 of the present invention. The sludge is treated, with any excess leachate passing through leachate drainage pipes 109 back to the waste water treatment facility. After a period of time the treated sludge, bedding combination can be harvested.

In this third embodiment the external box structure of the housing 50 is supported by a galvanised steel frame with panels of insulated fibre-glass so as to form an insulated housing 100. The housing 100 comprises walls 101 and a supporting floor 102. The housing also includes a roof 103. The interior of the housing 100 may be divided into a plurality of horizontally divided compartments by a partition floor system 104. Access to the housing 100 is via hinging doors 105. A control panel 1()6 housing electronic circuitry and key-pad access may be located on one of the gable ends 107, the control panel 106 may also incorporate the temperature sensor 45, moisture sensor 46 and pH sensor 47 described with reference to the second embodiment. Space heaters 41 may also be located on the gable end 107.

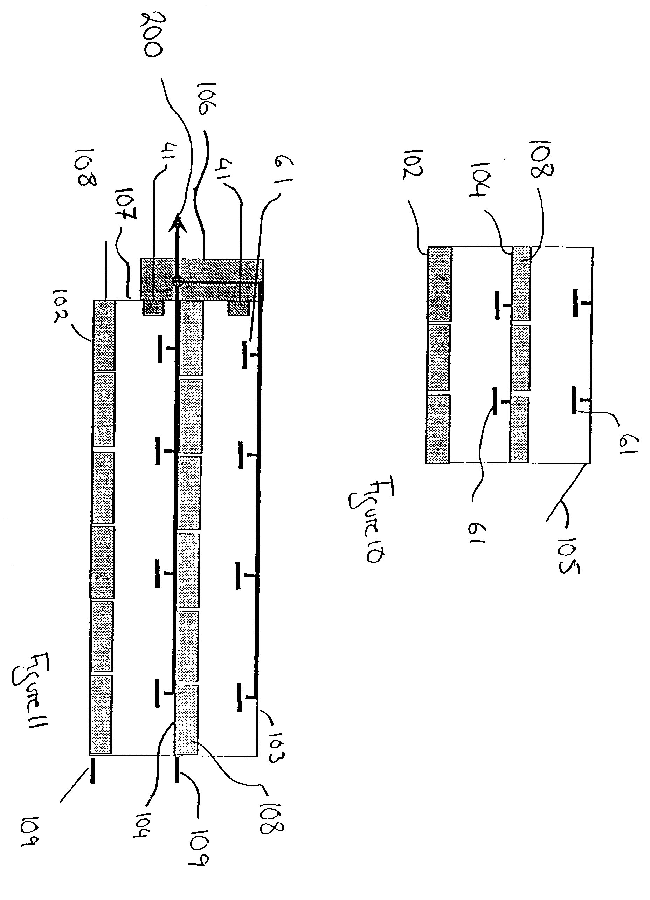

A typical enclosure houses a population of the compost worm Eisenia fetida (Oligochaeta) in a bedding of graded wood chips. Other redworm species may also be used for this purpose. In order to facilitate handling and harvesting, the worms 7 and wood chip bedding 55 material are transported to the site in plastic mesh trays 108. These are typically between 20 and 60 litres capacity and are stacked tightly into the housing on both the base floor 102 of the unit and the partition floor 104. A typical arrangement of the empty trays is shown in figures 10 and 11. The stocking density of worms is between eight and 10 kg/m2 and the wood chip used is of a soft wood conifer variety. The wood chip can be graded so that the maximum particle size of the finished product can be controlled.

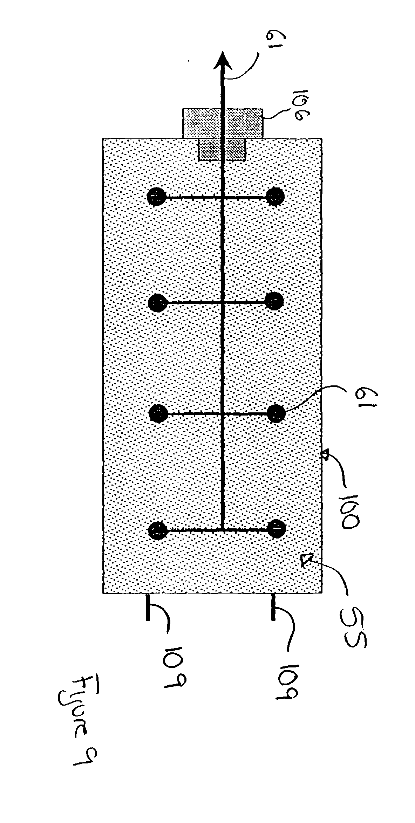

This third embodiment also includes applicator means such as described previously with reference to the second embodiment. The applicator means in fluid communication with the sludge settlement tank comprises plastic piping 61 connected to a submersible pump (not shown) in the sludge settlement zone of the associated waste water treatment plant (WWTP) 200. The submersible pump should contain a robust shredding device, such as the pyranha pump 60, in order to avoid clogging with non-sludge solids such as rags. This piping is connected to a sprinkler system 61 within the unit, which is arranged in order to deliver an even spread of sludge onto the worm bedding, as shown in figure 9. As the bedding and the plastic trays together act as an efficient filter, the unit is inclined and the leachate that gathers under the trays maybe passed from the worm housing, using a leachate drain system, through a further sand/vermicompost filter, housed in a separate adjoining container, downstream of the housing 100, prior to discharge to the WWTP.

The space heater 41 is thermostatically programmed to maintain the temperature within the optimum vermicomposting range, typically 18-22°C. In addition, the circulating air keeps the walls and roof of the unit dry, thus inhibiting the movements of the worms out of the bedding. For temperate climate applications, there is rarely a need to cool the system during periods of hot weather as the application of sludge will in itself have a cooling effect. However, in more tropical climates, where cooling will be necessary, the space heaters can be replaced with air-conditioning units.

A micro-controller 110 facilitates the automatic programming of the unit through a keyboard interface in the control panel 100. The main functions of the micro-controller are to programme the pumping rate and to dictate the temperature range of the thermostat. Additional features of the system may include data logging of sludge loading and temperature fluctuation. An alarm system may also be incorporated in the event of pump or heater failure. This alarm may be transmitted by modem (not shown) to a central control location. The modem link also allows for remote control of the unit's temperature and pumping programme along with a data interrogation facility. These features allow for the networking of multiple housing units.

Once the trays and worms have been suitably positioned with the housing 100, they may be left unattended for at least 6 months. The housing 50 facilitates sludge application and the control of the temperature regime. The system is designed to be directly connected to the sludge settlement zone of any primary and/or secondary WWTP by a submersible sludge pump. Liquid sludge in waste water treatment plants is typically a liquid with less than 5% solids per volume. The pump 60 is electronically programmed to deliver a set quantity of settled sludge to the unit at timed intervals. This sludge, which apart from being processed through a shredder pump, goes through no intervening steps between the tanks and the unit is applied to the worm bedding 55 using a sprinkler system 58 in a liquid form of between 0.5 and 5% solids. It will be appreciated that the sludge does not need any pre-treatment and can be transferred to the unit automatically without the need for day to day sludge collection, pre-treatment and application, as what is typical using the methods and apparatus of the prior art.

The wood chip bedding acts as an efficient filter with significant de-watering occurring within hours of sludge application. The de-watered sludge retained in the bedding undergoes the vermicomposting process. This involves further de-watering due to the constant turning of the material by the worm herd. Moreover, the sludge solids are ingested by the worms. This results in a dramatic decrease in enteric pathogen numbers, with parallel increases in aerobic non-pathogenic microbes in the egested material called castings. These beneficial microbes compost the remaining organic material into a stabilised non-fermentable product, i.e. off-odours are eliminated and enteric pathogens are reduced further. The process is a continual process with sludge being applied at regular intervals over a given period, typically between 6 and 18 months. By continual is meant any process which occurs regularly over a given period of time.

After this period of 6 to 18 months harvesting of the trays can take place. This involves the separation of the worms from the bedding. In the first and second embodiment, this had to occur after the bedding or IBCs were removed. Harvesting in these cases therefore involved either sieving the compost or preferably by drawing the worm population out of the compost by laying feed adjacent to the bedding at a holding location. In the case of the IBCs, these would have to be emptied out into the holding area to facilitate this separation. In the third embodiment however, a method has been developed that allows for this separation to be achieved within the housing, prior to harvesting, thus greatly expediating the process. This is achieved by placing a second layer of mesh trays containing wood chip only over the existing trays, by now full of vermicompost and worms. The positioning of the second tray interrupts the application of the sludge to the lowermost tray, and as sludge application continues as normal, after a period of approximately six weeks, the bulk of the worm population will have migrated, through the mesh layer, into the upper layer, leaving the lower trays virtually free of worms. At this stage, all of the trays are removed from the unit, with the upper trays containing the worm population being returned, while the lower trays are emptied into bags for storage prior to disposal. A storage period of at least two months is recommended in order for pathogen levels to reach low levels and to allow the vermicompost to dry further. In the absence of further sludge additions, the aerobic microbial population becomes fully established to the detriment of enteric pathogens in the period following harvesting. Moreover, as the vermicompost is no longer in constant receipt of wet sludge, it readily dries from an initial solids content of between 10 and 15% to a value in excess of 25%. This will occur under ambient conditions provided that precipitation is excluded.

After this two month ageing process, the vermicompost typically has a solids content greater than 25%. The texture and maximum particle size will be determined by the grading of the wood chip used at the outset. All off-odours associated with anaerobic conditions will have been eliminated as the product will be aerobic. The pathogenic bacterial content will be low generally with Salmonella species not being detected, and E. coli numbers of less than 100 MPN/g (MPN: most probable number).

It should be noted that the aerobic conditions in the vermicompost are facilitated by the continual turning of the sludge by worms within the vessel thus maintaining good levels of oxygen. This allows aerobic microbes to proliferate. Under these conditions, composting by-products include non-odourous compounds such as carbon dioxide, sulphates and nitrates. Conversely if anaerobic bacteria are allowed to proliferate, the following odourous gases among others are produced: methane, hydrogen sulphide and ammonia. Subsequently, when the sludge has been largely composted within the vessel, the remaining organic material will not be subject to the recolonisation of anaerobic species when the earthworms have been removed, i.e. the readily putrefiable fraction has been eliminated.

As described above, and shown in block format in Figure 8, it is envisaged that the invention will be incorporated into an existing sewage plant facility or waste treatment plant facility. Such facilities already have primary and secondary settling tanks. The shredder pump would be placed at the bottom of one or other of the sludge settlement tanks and the pump shreds it further and then pumps it to the unit of the present invention. Any liquid that filters through the bedding enclosures passes through the leachate exit, which is shown in Figure 10, and back into the primary settling tank.

There is thus provided by the invention a novel system for disposal of waste material, in which the waste is effectively consumed by worms living in a base structure, the environment of the system being controlled. The use of control means aids in the continual and optimum operation of the system. The invention allows for the application of a sludge material directly to the vermicomposting beds without a pre-treatment drying step. The use of the shredding mixing machine in conjunction with the worm beds allows bulkier waste material to be treated by vermiculture systems, than previously allowed.

The words "comprises/comprising" and the words "having/including" when used herein with reference to the present invention are used to specify the presence of stated features, integers, steps or components but does not preclude the presence or addition of one or more other features, integers, steps, components or groups thereof.

## Claims

1. A waste treatment system for the conversion of organic sludge into worm castings comprising:
i) a housing having a support floor, walls and a roof, the housing adapted to contain a fibrous worm bedding material adapted to sustain worms,
ii) applicator means in fluid communication with a sludge settlement tank for applying sludge from the settlement tank directly to the worm bedding material wherein the applicator means is adapted to apply the sludge continually to the bedding and the bedding material being removable to effect harvesting of the castings.

2. The waste treatment system as claimed in claim 1 wherein the bedding material is containable in a removeable bedding enclosure, the bedding enclosure being removeable to effect harvesting of the castings.

3. The waste treatment system as claimed in claim 2 wherein a plurality of removeable bedding enclosures are provided within the housing

4. The waste treatment system as claimed in any preceding claim wherein the applicator means includes a pump for pumping the sludge from the settlement tank to the bedding enclosure, and spray means for spraying the sludge onto the worm bedding material, such that the sludge being sprayed is in a liquid form with less than about 5% solid material per volume.

5. The waste treatment system according to any one of claims 2 to 4 wherein the bedding enclosure is a mesh tray, the tray being situatable on a support floor within the enclosure.

6. The waste treatment system as claimed in anyone of claims 2 to 5 wherein the housing comprises a plurality of floors, and a plurality of spray means, each floor adapted to support a plurality of bedding enclosures, and the spray means adapted for spraying the sludge material onto the bedding enclosures.

7. The waste treatment system as claimed in any preceding claim wherein the worm bedding material is adapted to provide a home for a worms, preferably red worms, and more preferably worms of the variety *Eisenia fetida.*

8. The waste treatment system as claimed in any one of claims 2 to 7 wherein the bedding enclosures are provided with mounting means, suitable for mounting an additional bedding enclosure containing worm bedding material on top of an existing bedding enclosure containing worms, such that the mounting of a bedding enclosure on top of the worm containing bedding enclosure interrupts the application of sludge from the applicator means onto the worm containing bedding enclosure, and stimulates the movement of worms from the lowermost bedding enclosure to the uppermost bedding enclosure.

9. The waste treatment system as claimed in any one of claims 2 to 8 wherein the bedding enclosures are provided with transport means, suitably apertures, for allowing movement of worms from a first bedding enclosure to a second bedding enclosure located above the first enclosure, the transport means being located in the base of the bedding enclosures, and also adapted to facilitate the passage of leachate onto the housing floor.

10. The waste treatment system as claimed in any preceding claim wherein the support floor is provided with a draining means for draining excess fluid that filters through the bedding material, the draining means in fluid communication with a waste water treatment plant, such that excess fluid is passed from the waste treatment system to the waste water treatment plant.

11. The waste treatment system as claimed in any preceding claim wherein the housing is insulated.

12. The waste treatment system as claimed in any preceding claim further comprising means for controlling at least two environmental parameters within the enclosure, the means being responsive to at least moisture level and temperature, and optionally light and pH level.

13. A method of converting organic sludge material to worm castings comprising the steps of:
a) providing a worm bedding material within a housing,
b) housing worms within said worm bedding material, and
c) applying organic sludge material directly from a sludge settlement tank to the bedding material.

14. The method as claimed in claim 13 comprising the additional step of placing the bedding material into a plurality of bedding enclosures prior to the housing of the worms in the bedding material.

15. The method as claimed in claim 13 or 14 wherein the sludge is applied to the surface of the bedding material in a liquid form with less than 5% solids per volume.

16. The method as claimed in any one of claims 13 to 15 wherein the step of positioning the bedding enclosure within the housing comprises the suspension of the bedding enclosure within the housing or mounting of the bedding enclosure on a floor within the housing.

17. The method as claimed in any one of claims 13 to 16 comprising the additional step of mounting a second bedding enclosure on top of the first bedding enclosure, the mounting of the second bedding enclosure stimulating the movement of worms from the first bedding enclosure to the second bedding enclosure.

18. The method as claimed in any one of claims 13 to 17 comprising the additional step of removing the bedding enclosures comprising bedding material and worm castings from the housing at most once every 6 months.
